# EUROPEAN PATENT APPLICATION

(11) **EP 2 689 715 A1**
(43) Date of publication of application: **29.01.2014**
(21) Application number: 13743707.5
(22) Date of filing: 08.01.2013
(51) Int. Cl.: A61B 1/00

(54) **INSERTION DEVICE**

(30) Priority: 30.01.2012 JP 2012017219
(71) Applicant: Olympus Medical Systems Corp., Tokyo 151-0072 (JP)
(72) Inventor: OKAMOTO, Yasuhiro, Tokyo 151-0072 (JP)
(74) Representative: Schicker, Silvia
(86) International application number: PCT/JP2013/050085
(87) International publication number: WO 2013/114913

(57) **Abstract**

An insertion portion, an operation portion 5, a pull wire 10, a joy stick 15, and a guide roller 14 that is provided outside an action range of a frame body 11 that acts by being linked with tilt of an operation element 8 and a shaft 9 in the operation portion 5, and changes a travel direction of the pull wire 10 that is extended in a height direction Z from the frame body 11 so that the pull wire travels outside the action range of the frame body 11 to a front side in an insertion direction S.

## Description

### Technical Field

The present invention relates to an insertion apparatus that includes an insertion portion that is inserted into a subject, with a bending portion that bends in a plurality of directions being provided in the insertion portion.

### Background Art

In recent years, insertion apparatuses, for example, endoscopes having insertion portions that are inserted into subjects have been widely used in medical fields and industrial fields.

The endoscopes for use in the medical fields are capable of observing organs in body cavities and performing various treatments with use of treatment instruments inserted into insertion channels for the treatment instruments that are included by the endoscopes in accordance with necessity, by elongated insertion portions being inserted into body cavities to be subjects.

Further, the endoscopes for use in the industrial fields are capable of performing inspection such as observation of flaws, corrosion and the like of sites to be examined in objects and various treatments by elongated insertion portions of the endoscopes being inserted into the objects such as jet engines and piping of factories.

Here, a configuration is known, in which a bending portion bendable in a plurality of directions is provided at a distal end side in the insertion direction of the insertion portion of an endoscope (hereinafter simply referred to as a distal end side).

More specifically, to the bending portion, a distal end in the insertion direction (hereinafter simply referred to as a distal end) of a pull wire that is a pull member inserted through the insides of the insertion portion and the operation portion is connected, and the pull wire is operated to be pulled by an operation input member such as a joystick provided in the operation portion, whereby the bending portion is bendable in a plurality of directions.

Here, the configuration of an endoscope having an electric assist mechanism that renders assistance in the pulling operation of a bending wire by electric power is also known, and is disclosed in, for example, Japanese Patent Application Laid-Open Publication No. 2004-321492.

The electric assist mechanism disclosed in Japanese Patent Application Laid-Open Publication No. 2004-321492 has the configuration in which a pulley that is a drive section rotating in one direction by a motor or the like is included at a lower side of a frame body to which the proximal end of the pull wire of a joystick in an operation portion is connected, and the pull wire at an intermediate position is wound around the outer periphery of the pulley in a slack state.

The electric assist mechanism is configured so that when the pull wire is pulled by the joystick, the pull wire is reduced in diameter to be brought into contact with the pulley with a frictional force, and rotates in one direction with the pulley, whereby a pull assisting force is given to the pull wire by the rotation.

However, in the configuration disclosed in Japanese Patent Application Laid-Open Publication No. 2004-321492, the electric assist mechanism is provided below the joystick in the operation portion and it is configured that the pull wire is extended from the frame body of the joystick toward the pulley of the electric assist mechanism by a predetermined length. Thus, there has been a problem that a size of the operation portion is increased in a direction connecting the joystick and the pulley.

The present invention has been made in view of the above circumstances and an object of the present invention is to provide an insertion apparatus capable of achieving downsizing of an operation portion in a configuration that an electric assist mechanism for giving a pull assisting force to a pull member which operates to bend a bending portion is provided in the operation portion.

### Disclosure of Invention

### Means for Solving the Problem

An insertion apparatus according to one aspect of the present invention includes an insertion portion that is inserted into a subject, and is provided with a bending portion that bends in a plurality of directions, an operation portion that is provided at a proximal end in an insertion direction of the insertion portion, a pull member that is inserted through insides of the insertion portion and the operation portion, has a distal end in the insertion direction connected to the bending portion, and causes the bending portion to bend by pulling, an operation input member that is provided in the operation portion, has a shaft that is raised in an intersection direction intersecting the insertion direction, an operation element provided at an upper end portion of the shaft and a connecting portion that is provided at a lower end portion of the shaft and to which a proximal end of the pull member in the insertion direction is connected in the operation portion, in which input of a pulling operation of the pull member is performed by the operation element and the shaft being tilted and the connecting portion being brought into action, and a travel direction changing member that is provided outside an action range of the connecting portion that acts by being linked with tilt of the operation element and the shaft in the operation portion, and changes a travel direction of the pull member extended in the intersection direction from the connecting portion so that the pull member travels outside the action range of the connecting portion to a front side in the insertion direction.

### Brief Description of the Drawings

Fig. 1 is a perspective view schematically showing an endoscope showing a present embodiment;
Fig. 2 is a top view of an electric assist mechanism of Fig. 1 seen from a II direction in Fig. 1;
Fig. 3 is a side view schematically showing the electric assist mechanism of Fig. 2 seen from a III direction in Fig. 2;
Fig. 4 is a view of a joystick of Fig. 3 seen from a IV direction in Fig. 3;
Fig. 5 is a partial sectional view showing a modification in which a protection member is provided at a position of a pull wire that is superimposed on the joystick at a time of the protection member being planarly viewed from a height direction, in an operation portion of Fig. 3;
Fig. 6 is a view showing a holding mechanism provided at a lower side of a frame body of the joystick in the operation portion of Fig. 3;
Fig. 7 is a view schematically showing an electric assist mechanism, showing a modification in which a guide roller of Fig. 3 is provided at a lower side of the frame body of the joystick;
Fig. 8 is a view showing a holding mechanism and the joystick of Fig. 7 under enlargement;
Fig. 9 is a partial sectional view taken along a IX-IX line in Fig. 8; and
Fig. 10 is a view showing a modification in which travel direction changing members are configured by cylindrical members through which the pull wires at positions that are superimposed on the joystick at the time of the travel direction changing members being planarly viewed from the height direction are inserted in the operation portion of Fig. 3.

### Best Mode for Carrying Out the Invention

Hereinafter, an embodiment of the present invention will be described with reference to the drawings. Note that the drawings are schematic, attention should be paid to that the relationships of the thicknesses and the widths of the respective members, the ratios of the thicknesses of the respective members and the like differ from the actual relationships, ratios and the like, and among the drawings, the parts in which the mutual relationships and ratios of the dimensions differ are included as a matter of course. Note that hereinafter, in the present embodiment, an insertion apparatus will be described with a medical apparatus, for example, an endoscope cited as an example.

Hereinafter, an embodiment of the present invention will be described with reference to the drawings.

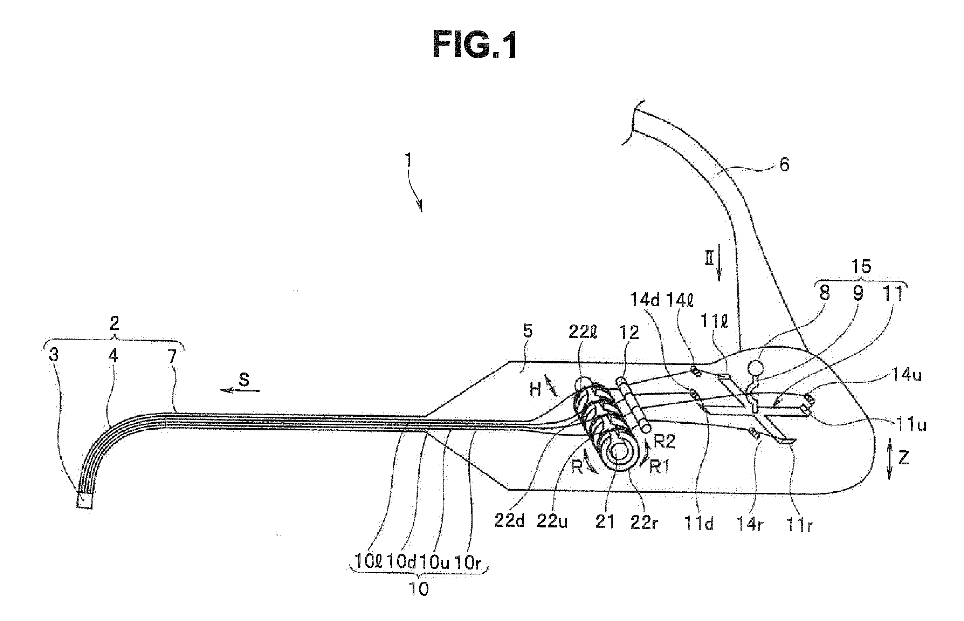

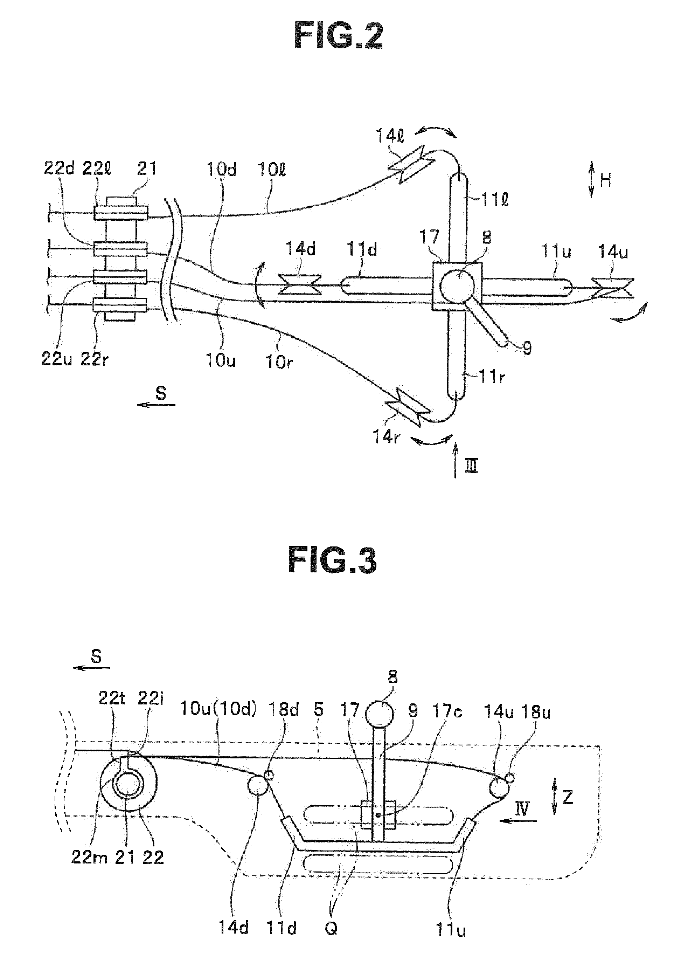

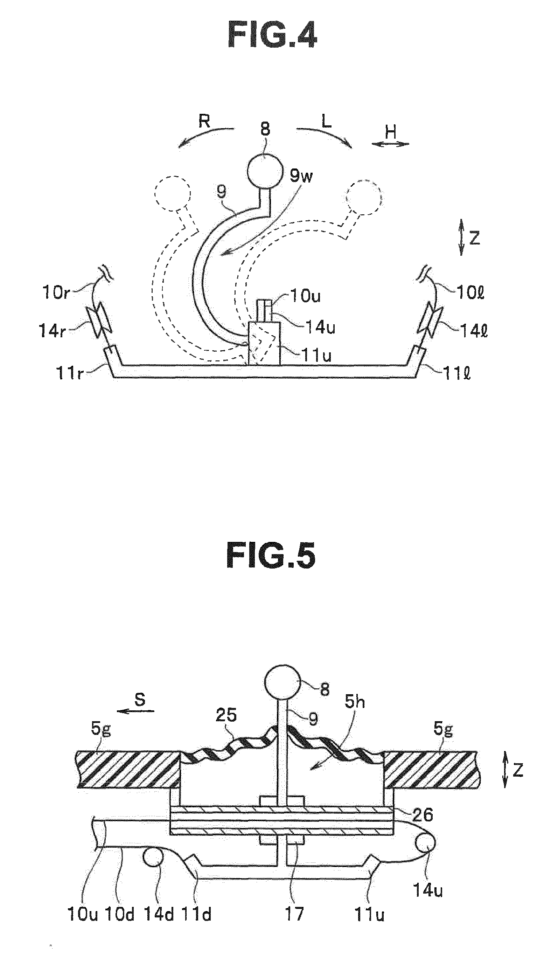

Fig. 1 is a perspective view schematically showing an endoscope showing the present embodiment. Fig. 2 is a top view of an electric assist mechanism of Fig. 1 seen from a II direction in Fig. 1. Fig. 3 is a side view schematically showing the electric assist mechanism of Fig. 2 seen from a III direction in Fig. 2. Fig. 4 is a view of a joystick of Fig. 3 seen from a IV direction in Fig. 3.

As shown in Fig. 1, an endoscope 1 has a main part configured by including an insertion portion 2 that is inserted into a subject, an operation portion 5 connected to a proximal end in an insertion direction S of the insertion portion 2 (hereinafter simply referred to as a proximal end), and a universal cord 6 extended from the operation portion 5, and is connectable to a peripheral apparatus via a connector not illustrated and provided at an extension end of the universal cord 6.

The insertion portion 2 has a main part configured by a distal end portion 3, a bending portion 4 and a flexible tube portion 7, and is formed to be elongated along the insertion direction S.

The bending portion 4 is operated to bend in a plurality of directions, for example, four directions, up and down, and left and right by a joystick 15 that will be described later and is provided at the operation portion 5 being operated.

When a bending configuration of the bending portion 4 is briefly described, pull wires 10u, 10d, 10r and 101 (hereinafter collectively referred to as pull wires 10) that are a plurality of, for example, four pull members are inserted through insides of the insertion portion 2 and the operation portion 5 respectively by being displaced by 90° from one another in a circumferential direction of the insertion portion 2, and distal ends of the respective pull wires 10u, 10d, 10r and 101 are connected to the bending portion 4.

The pull wire 10u causes the bending portion 4 to bend in an upper direction by being pulled by following a tilting operation to the upper direction by the joystick 15. The pull wire 10d causes the bending portion 4 to bend in a lower direction by being pulled by following a tilting operation to the lower direction by the joystick 15.

Further, the pull wire 10r causes the bending portion 4 to bend in a right direction by being pulled by following a tilting operation to the right direction by the joystick 15. Further, the pull wire 101 causes the bending portion 4 to bend in a left direction by being pulled by following a tilting operation to the left direction by the joystick 15.

The operation portion 5 is provided with the joystick 15 that is an operation input member in which input of the pulling operation of the pull wire 10 is performed.

The joystick 15 has a main part configured by having a shaft 9 that is provided to be raised in a height direction Z that is an intersection direction intersecting the insertion direction S substantially perpendicularly, for example and extend to an outside of the operation portion 5 from the inside of the operation portion 5, an operation element 8 that is provided at an upper end portion in the height direction Z of the shaft 9 that is located in the outside of the operation portion 5, and a frame body 11 that is a connecting portion provided at a lower end portion in the height direction Z of the shaft 9 that is located in the operation portion 5.

In the joystick 15, the operation element 8 and the shaft 9 are tiltable in the four directions, up and down, and left and right with a rotation center 17c as a starting point by, for example, a universal joint 17 provided at a lower side of the shaft 9 as shown in Fig. 3.

Accordingly, the joystick 15 is for performing input of a pulling operation of the pull wire 10 by the operation element 8 as well as the shaft 9 being tilted by an operator and the frame body 11 being brought into action.

Note that the frame body 11 is formed by having a shape of a cross when the frame body 11 is planarly viewed from the height direction Z. A proximal end of the pull wire 10u is connected to an extension end of an arm portion 11u that is located to extend to a rear side along the insertion direction S. A proximal end of the pull wire 10d is connected to an extension end of an arm portion 11d that is located to extend to a front side along the insertion direction S. A proximal end of the pull wire 10r is connected to an extension end of an arm portion 11r that is located to extend to one side along a width direction H that is orthogonal to the insertion direction S and the height direction Z. A proximal end of the pull wire 101 is connected to an extension end of an arm portion 111 that is located to extend to the other side along the width direction H.

By the above, such a configuration is provided that when the operation element 8 and the shaft 9 are tilted in the upper direction and the arm portion 11u is brought into action, the pull wire 10u the proximal end of which is connected to the extension end of the arm portion 11u is pulled to a rear side in the insertion direction S (hereinafter simply referred to as a rear side), when the operation element 8 and the shaft 9 are tilted in the lower direction and the arm portion 11d is brought into action, the pull wire 10d the proximal end of which is connected to the extension end of the arm portion 11d is pulled to the rear side, when the operation element 8 and the shaft 9 are tilted in the right direction and the arm portion 11r is brought into action, the pull wire 10r the proximal end of which is connected to the extension end of the arm portion 11r is pulled to the rear side, and when the operation element 8 and the shaft 9 are tilted in the left direction and the arm portion 111 is brought into action, the pull wire 101 the proximal end of which is connected to the extension end of the arm portion 111 is pulled to the rear side.

Further, in the operation portion 5, in vicinities of upper sides in the height direction Z (hereinafter simply referred to as an upper side) of the extension ends to which the proximal ends of the respective pull wires 10u, 10d, 10r and 101 are connected, of the respective arm portions 11u, 11d, 11r and 111 of the frame body 11, outside action ranges of the respective arm portions 11u, 11d, 11r and 111 that act by being linked with the tilt of the operation element 8 and the shaft 9, guide rollers 14u, 14d, 14r, and 141 that are travel direction changing members that change travel directions of the pull wires 10u, 10d, 10r and 101 that are extended to the upper side from the extension ends of the respective arm portions 11u, 11d, 11r and 111 so that the pull wires travel to a front side in the insertion direction S (hereinafter simply referred to as a front side) outside the action ranges of the respective arm portions 11u, 11d, 11r and 111 are provided to correspond to the pull wires 10u, 10d, 10r and 101.

Note that the action ranges of the respective arm portions 11u, 11d, 11r and 111 in the present embodiment refer to regions where the respective arm portions 11u, 11d, 11r and 111 act, in a region Q in a lower side in the height direction Z of the frame body 11 (hereinafter simply referred to as a lower side) or in the upper side as shown in Fig. 3.

Further, the guide rollers 14u, 14d, 14r and 141 may be respectively provided in a region that is superimposed on the frame body 11 when the guide rollers are planarly viewed from the height direction Z.

The pull wires 10u, 10d, 10r and 101 are extended straight forward along the insertion direction S toward C rings 22u, 22d, 22r and 221 that will be described later from the guide rollers 14u, 14d, 14r and 141.

Further, the guide rollers 14u, 14d, 14r and 141 may be movable in a planar direction when the guide rollers are planarly viewed from the height direction Z as shown in Fig. 2 by following the pull wires 10u, 10d, 10r and 101 in response to tension directions of the pull wires 10u, 10d, 10r and 101.

Furthermore, as shown in Fig. 3, in the operation portion 5, in the vicinities of the guide rollers 14u, 14d, 14r and 141, stoppers 18u, 18d, 18r and 181 (the stoppers 18r and 181 are not illustrated) that prevent the pull wires 10u, 10d, 10r and 101 from being detached from the guide rollers 14u, 14d, 14r and 141 are provided.

Further, in the operation portion 5, a pulley 21 that is a drive member and the C rings 22u, 22d, 22r and 221 (hereinafter collectively referred to as C ring 22) that are drive force transmitting members are provided at the front side from the joystick 15.

More specifically, the pulley 21 generates a drive force that drives and bends the bending portion 4, is formed into an elongated columnar shape along the width direction H of the operation portion 5, and is configured to rotate in one direction R1 of rotational directions R at all times by a motor not illustrated when a power supply of the endoscope 1 is on.

Note that the pulley 21 is provided in a position at the same height as the guide rollers 14u, 14d, 14r and 141 in the height direction Z.

Four of the C rings 22 are provided as the C rings 22r, 221, 22d and 22u by being separated respectively along the width direction H, in an outer periphery of the pulley 21, and are located at the outer periphery of the pulley 21 in a noncontact manner.

Further, the pull wires 10r, 101, 10d and 10u in intermediate positions are respectively wound around outer peripheries of the respective four C rings 22r, 221, 22d and 22u.

More specifically, as shown in Fig. 3, the pull wire 10 that is extended to the rear side from the bending portion 4 is wound around an outer periphery of the C ring 22 by being wound from one end 22i side in a notch formed in the C ring 22 along the outer periphery of the C ring 22, and thereafter being extended along the insertion direction S to the rear side from the other end 22t side.

Thereby, a distal end side of the pull wire 10 is configured to be pulled to the rear side when the C ring 22 is rotated in the one direction R1, and to be slacked to the front side when the C ring 22 is rotated in the other direction R2 to an opposite side from the one direction R1.

Note that the C ring 22u may be a C ring shared by the C ring 22d, and the pull wires 10u and 10d may be wound around the shared C ring. Further, the C ring 22r may be a C ring shared by the C ring 221, and the pull wires 10r and 101 may be wound around the shared C ring.

Here, the guide roller 14u is provided at the upper side from the frame body 11, and the pulley 21 and the guide roller 14u are provided at the same height. For this reason, the pull wire 10u that is extended straight forward along the insertion direction S via the guide roller 14u from the extension end of the arm portion 11u to the C ring 22u is likely to contact the shaft 9.

However, the contact with the shaft 9 is avoided by the shaft 9 having a bent shape having a bent portion 9w in an intermediate position in the height direction Z, more specifically, a position between the operation element 8 and the universal joint 17 in the height direction Z so as to avoid interference of the pull wire 10u, as shown in Fig. 4. Namely, the pull wire 10u is inserted through a space formed by the bent portion 9w.

Note that the bent portion 9w is formed into such a shape that the pull wire 10u does not contact the shaft 9 even when the operation element 8 and the shaft 9 are tilted in a right direction (R) and a left direction (L) as shown by the dotted lines of Fig. 4.

From the above, the pull wire 10u can be extended straight forward along the insertion direction S without bypassing the shaft 9 from the extension end of the arm portion 11u to the C ring 22u by the bent portion 9w.

The C rings 22u, 22d, 22r and 221 transmit a rotational force in the one direction R1 that is a drive force of the pulley 21 and give a pull assisting force, to the pull wires 10u, 10d, 10r and 101 by rotating in the one direction R1 with the pulley 21 by a friction engaging portion 22m being frictionally engaged with the pulley 21 with a frictional force with the pull wires 10u, 10d, 10r and 101 being pulled to the rear side by the input operation of the joystick 15.

More specifically, when the pull wire 10u is pulled, for example, as the operation element 8 of the joystick 15 is tilted in the upper direction, the C ring 22u is reduced in diameter, and the friction engaging portion 22m contacts the outer periphery of the pulley 21 with a frictional force.

Accordingly, the C ring 22u gives a pull assisting force to a distal end side of the pull wire 10u by rotating in the one direction R1 with the pulley 21. As a result, the bending portion 4 is bent in the upper direction.

Note that since at this time, the C rings 22r, 221 and 22d do not contact the outer periphery of the pulley 21, the pull wires 10r, 101 and 10d are not pulled.

Further, the above pulling action is similarly applied to the C rings 22r, 221 and 22d, and is configured to cause the bending portion 4 to bend in the right, the left and the down directions by using the fact that when the pull wires 10r and 101 are pulled as the operation element 8 of the joystick 15 is tilted in the right, the left and the down directions, the C rings 22r, 221 and 22d are reduced in diameter, and contact the outer periphery of the pulley 21 with the frictional force.

Note that in the present embodiment, the electric assist mechanism is configured by the pulley 21, the C ring 22, the guide rollers 12, 14u, 14d, 14r and 141 and the joystick 15 that are described above.

As above, in the present embodiment, it is shown that the pulley 21 that generates the drive force that drives and bends the bending portion 4, and the C ring 22 that transmits the drive force of the pulley 21 to the pull wire 10 are provided at the front side from the joystick 15 so that the pull wires 10u, 10d, 10r and 101 are extended straight forward along the insertion direction S towards the C rings 22u, 22d, 22r and 22 from the respective guide rollers 14u, 14d, 14r and 141 in the operation portion 5.

Further, it is shown that the guide rollers 14u, 14d, 14r and 141 that change the travel directions of the pull wires 10u, 10d, 10r and 101 that are extended to the upper side from the respective arm portions 11 u, 11d, 11r and 11l of the frame body 11 of the joystick 15 to the front side are provided in the vicinities of the upper sides of the respective extension ends of the respective arm portions 11u, 11d, 11r and 11l in the height direction Z.

According to the above, the pull wires 10u, 10d, 10r and 101 are not extended to the lower side from the respective arm portions 11 u, 11d, 11r and 11l of the frame body 11 as in the prior art, and therefore, the operation portion 5 can be made compact in the height direction Z.

Further, the guide rollers 14u, 14d, 14r and 141 are provided in the positions in the vicinities of the extension ends of the respective arm portions 11u, 11d, 11r and 11l, and therefore, in the operation portion 5, the guide rollers 14u, 14d, 14r and 141 can be disposed with the space saved in the height direction Z.

Further, the guide rollers 14u, 14d, 14r and 141 are provided at the upper side from the respective arm portions 11u, 11d, 11r and 11l, and thereby located outside the action range of the respective arm portions 11u, 11d, 11r and 11l, and therefore, the guide rollers 14u, 14d, 14r and 141 do not hinder action of the respective arm portions 11u, 11d, 11r and 11l.

Furthermore, the guide rollers 14u, 14d, 14r and 14l change the travel directions of the pull wires 10u, 10d, 10r and 101 to be outside the action ranges of the respective arm portions 11u, 11d, 11r and 11l, and therefore, the pull wires 10u, 10d, 10r and 101 do not hinder action of the respective arm portions 11u, 11d, 11r and 11l.

From the above, the endoscope 1 can be provided, which can realize reduction in size of the operation portion 5, in the configuration in which the electric assist mechanism that gives the pull assisting force to the pull wire 10 that operates and bends the bending portion 4 is provided in the operation portion 5.

Note that hereinafter, a modification will be shown with use of Fig. 5. Fig. 5 is a partial sectional view showing a modification in which a protection member is provided in a position of a pull wire that is superimposed on the joystick when the protection member is planarly viewed in the height direction, in the operation portion of Fig. 3.

As shown in Fig. 5, in an outer sheath member 5g of the operation portion 5, in a site in which the shaft 9 of the joystick 15 is extended to an outside of the operation portion 5 from the inside of the operation portion 5 along the height direction Z, an opening 5h for causing the shaft 9 to extend to the outside of the operation portion 5 is formed, and the opening 5h has a configuration in which a rubber sheet 25 is provided to secure water tightness of the inside of the operation portion 5.

However, if the rubber sheet 25 is pushed into the operation portion 5 by a finger or the like of an operator, the rubber sheet 25 presses, for example, the pull wire 10u, and the pull wire 10u is likely to be pulled unintentionally.

Thus, as shown in Fig. 5, in the operation portion 5, an outer periphery of a location of the pull wire 10u that is superimposed on the joystick 15 when the location of the pull wire 10u is planarly viewed from the height direction Z, namely, the location of the pull wire 10u that is superimposed on the opening 5h may be covered with a rigid pipe 26 of stainless steel or the like that is a protection member protecting the pull wire 10u.

According to the above configuration, even if the rubber sheet 25 is pushed into the operation portion 5 with a finger or the like of the operator, the finger or the like of the operator contacts the rigid pipe 26, and therefore, does not press the pull wire 10u unintentionally.

Note that the above configuration is similarly applicable to the pull wires 10d, 10r and 10l, and the outer peripheries of the pull wires 10d, 10r and 101 may be covered with the pipe 26.

Further, the protection member is not limited to a pipe, but may be simply a plate, or may be a member made by a plate being bent, a casing or the like. Namely, any member may be adopted if only the member prevents the finger or the like of the operator that pushes the rubber sheet 25 from pressing the pull wires.

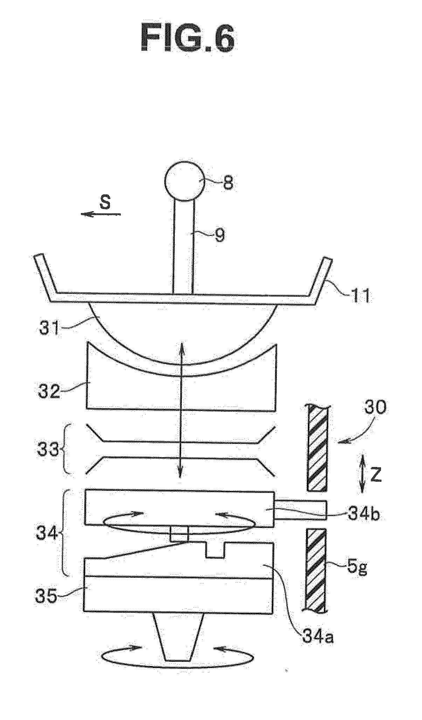

Further, hereinafter, another modification will be described with use of Fig. 6. Fig. 6 is a view showing a holding mechanism provided at the lower side of the frame body of the joystick in the operation portion of Fig. 3.

In the operation portion 5, in the region Q shown by the two-dot chain line at the lower side or the upper side in the height direction Z of the frame body 11 of the joystick 15 shown in Fig. 3, a holding mechanism 30 that changeably holds a tilt angle of the joystick 15 shown in Fig. 6 may be provided.

More specifically, the holding mechanism 30 has a main part configured by a semispherical pressed member 31 that is fixed to a bottom surface of the frame body 11, a pressing member 32 provided at a lower side of the pressed member 31, a spring 33 provided at a lower side of the pressing member 32, a cam member 34 provided at a lower side of the spring 33, and an engaging knob 35 provided at a lower side of the cam member 34.

The pressing member 32 presses the pressed member 31, and is formed into a shape in which the pressed member 31 is fitted.

The spring 33 presses the pressing member 32 toward the pressed member 31. The cam member 34 has a main part configured by a first cam 34a that has an inclined surface formed thereon and rotates with the engaging knob 35, and a second cam 34b that is movable in the height direction Z via the inclined surface of the first cam 34a with rotation of the first cam 34a in one direction or the other direction.

When the tilt angle of the operation element 8 and the shaft 9 in the joystick 15 is fixed with use of the holding mechanism 30 having the configuration as above, if the engaging knob 35 is rotated in one direction by the operator, the first cam 34a also rotates in the one direction, whereby the second cam 34b in contact with the inclined surface of the first cam 34a moves to the upper side.

Thereafter, the second cam 34b presses the spring 33 to the upper side, and thereby the pressing member 32 is pressed by the spring 33 and presses the pressed member 31. As a result, action of the frame body 11 is fixed, and therefore, the tilt angle of the operation element 8 and the shaft 9 is fixed.

Note that in a case of fixation of the tilt angle of the operation element 8 and the shaft 9 being released, when the engaging knob 35 is rotated in the other direction opposite from the one direction by the operator, the first cam 34a also rotates in the other direction, and thereby the second cam 34b that is in contact with the inclined surface of the first cam 34a moves to the lower side.

As a result, the second cam 34b moves to the lower side from the spring 33, and the pressing member 32 also moves to the lower side with respect to the pressed member 31, whereby fixation of the tilt angle of the operation element 8 and the shaft 9 is released.

Here, if in the operation portion 5, a mechanism that fixes the tilt angle of the joystick 15 by fixing movement of the pull wire 10, for example, is additionally provided at a position different from the position where the joystick 15 is provided, the site increases in size in the height direction Z more than the prior art.

However, according to the aforementioned configuration, if the holding mechanism 30 is provided in the vacant region Q at the lower side of the frame body 11 of the joystick 15, or the vacant region Q at the upper side, even when the holding mechanism for the tilt angle of the joystick 15 is provided in the operation portion 5, the operation portion 5 does not increase in size in the height direction Z.

Namely, reduction in size in the height direction Z of the operation portion 5 can be realized, and reduction in size of the operation portion 5 can be realized.

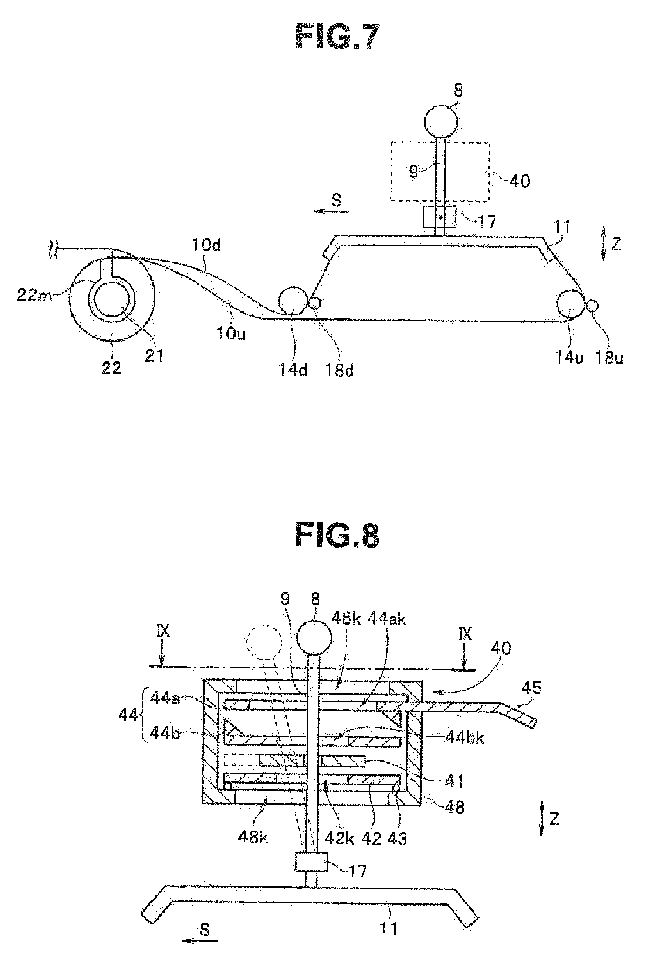

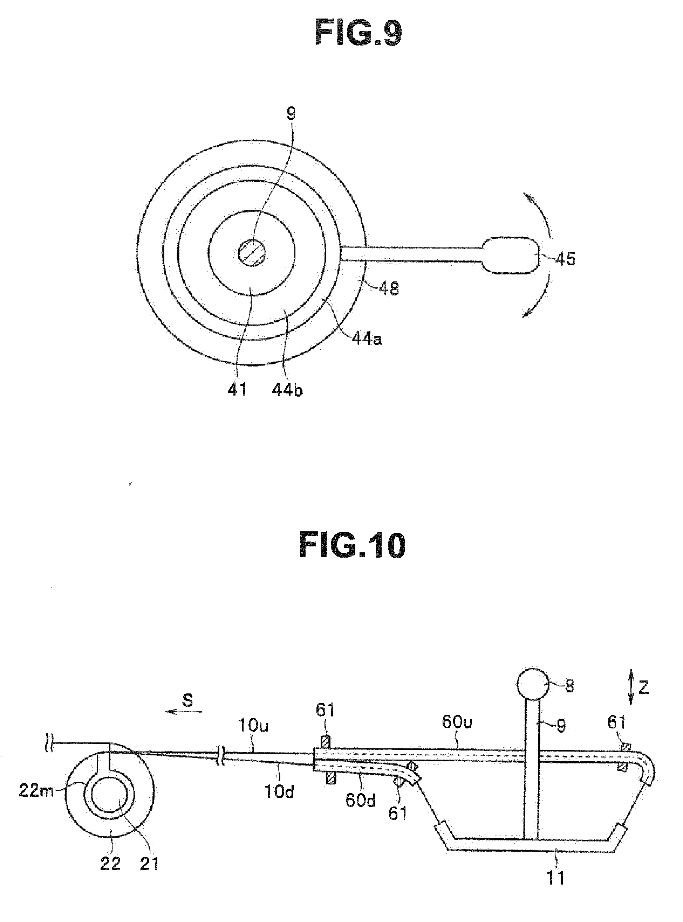

Further, hereinafter, another modification will be shown with use of Fig. 7 to Fig. 9. Fig. 7 is a view schematically showing an electric assist mechanism showing a modification in which the guide rollers of Fig. 3 are provided at the lower side from the frame body of the joystick. Fig. 8 is a view showing the holding mechanism and the joystick of Fig. 7 under enlargement. Fig. 9 is a partial sectional view taken along a IX-IX line in Fig. 8.

In the present embodiment described above, it is shown that the guide rollers 14u, 14d, 14r and 141 are provided in the positions in the vicinities of the upper sides of the extension ends of the arm portions 11u, 11d, 11r and 11l of the frame body 11.

The guide rollers 14u, 14d, 14r and 141 (note that in Fig. 7, the guide rollers 14r and 141 are not illustrated) are not restricted to the above, and may be provided at positions in the vicinities of lower sides of the extension ends of the arm portions 11u, 11d, 11r and 11l of the frame body 11 in the joystick 15, as shown in Fig. 7.

Note that in the configuration, the guide rollers 14u, 14d, 14r and 14l are also provided at positions out of the action range of the arm portions 11u, 11d, 11r and 11l.

Further, in the configuration as above, the guide rollers 14u, 14d, 14r and 141 have the functions of changing the travel directions of the pull wires 10u, 10d, 10r and 101 that are extended to the lower side from the extension ends of the arm portions 11u, 11d, 11r r and 11l to the front side so that the pull wires travel outside the action range of the arm portions 11u, 11d, 11r and 111.

Note that in the configuration shown in Fig. 7, in the operation portion 5, in the vicinities of the guide rollers 14u, 14d, 14r and 141, the stoppers 18u, 18d, 18r and 181 (the stoppers 18r and 181 are not illustrated) that prevent the pull wires 10u, 10d, 10r and 10l from being detached from the guide rollers 14u, 14, 14r and 141 are also provided. Further, the other configurations are the same as the configuration of the present embodiment described above.

According to the configuration as above, not only the similar effect as the effect of the present embodiment described above can be obtained, but also even when the rubber sheet 25 is pushed in with the finger or the like of the operator as described above, the finger or the like contacts the frame body 11, and therefore, does not press the pull wire 10, because the pull wire 10 is located at the lower side from the frame body 11.

Consequently, as shown in Fig. 5, the outer periphery of the pull wire 10 does not have to be covered with the protection member such as the pipe 26. Namely, in the configuration shown in Fig. 7, the frame body 11 configures the protection member.

However, in the configuration shown in Fig. 7, the operation portion 5 becomes larger in size in the height direction Z than in the present embodiment, correspondingly to the guide rollers 14u, 14d, 14r and 141 being provided at the lower side from the frame body 11.

Therefore, in the configuration of the electric assist mechanism shown in Fig. 7, reduction in size in the height direction Z of the operation portion 5 can be realized, if the holding mechanism that changeably holds the tilt angle of the joystick 15 described above is provided at an intermediate position in the height direction Z of the shaft 9 instead of the lower side of the frame body 11, as shown in Fig. 8 and Fig. 9.

More specifically, as shown in Fig. 8, a holding mechanism 40 that changeably holds the tilt angle of the operation element 8 and the shaft 9 of the joystick 15 is provided at the intermediate position in the height direction Z of the shaft 9.

The holding mechanism 40 has a main part configured by having a cam member 44, a pressed member 41 that is located at a lower side of the cam member 44, a pressing member 42 that is located at a lower side of the pressed member 41, and a spring 43 that is located at a lower side of the pressing member 42, that are provided in a frame 48 in which holes 48k through which the shaft 9 is inserted along the height direction Z are formed in a top surface and a bottom surface in the height direction Z.

The cam member 44 has a main part configured by a rotatable first cam 44a that has a hole 44ak through which the shaft 9 is inserted along the height direction Z formed therein, and is configured by a same member as an engage lever 45, and a second cam 44b that is located at a lower side of the first cam 44a, has a hole 44bk through which the shaft 9 is inserted along the height direction Z formed therein, and is movable along the height direction Z by rotation of the first cam 44a in one direction or the other direction. Note that the second cam 44b is formed to be larger in diameter than the pressed member 41.

The pressed member 41 is a member that is sandwiched by the second cam 44b and the pressing member 42 in the height direction Z, and is movable in the frame 48 in accordance with tilt of the shaft 9 by being fixed to the shaft 9.

The pressing member 42 is a member that is formed to be larger in diameter than the pressed member 41, and sandwiches the pressed member 41 between the pressing member 42 and the second cam 44b in the height direction Z, and has a hole 42k through which the shaft 9 is inserted along the height direction Z formed therein.

Note that the above described respective holes 48k, 44ak, 44bk and 42k are formed to have diameters larger than the shaft 9, more specifically, diameters of sizes in which the shaft 9 can tilt.

The spring 43 is configured by, for example, a disk spring, and is provided between the bottom surface of the frame 48 and the pressing member 42 in the height direction Z.

In a case in which the tilt angle of the operation element 8 and the shaft 9 in the joystick 15 is fixed with use of the holding mechanism 40 having the configuration as above, when the engage lever 45 is rotated in one direction by an operator, the first cam 44a also rotates in the one direction, and thereby the second cam 44b in contact with an inclined surface of the first cam 44a moves to the lower side.

Thereafter, the second cam 44b sandwiches the pressed member 41 that is movable with the shaft 9 between the second cam 44b and the pressing member 42 in the height direction Z, against an urging force to the upper side, of the spring 43, and thereby, the tilt angle of the operation element 8 and the shaft 9 is fixed.

Note that in a case in which fixation of the tilt angle of the operation element 8 and the shaft 9 is released, when the engage lever 45 is rotated in the other direction opposite from the one direction by the operator, the first cam 44a also rotates in the other direction, and thereby the second cam 44b that is in contact with the inclined surface of the first cam 44a moves to the upper side.

As a result, the second cam 44b separates from the pressed member 41 to the upper side in the height direction Z, and therefore, sandwiching by the pressing member 42 and the second cam 44b is released. Accordingly, the pressed member 41 becomes movable with the shaft 9, and thereby the fixation of the tilt angle of the operation element 8 and the shaft 9 is released.

According to the configuration as above, the pressed member 41 is sandwiched by the pressing member 42 and the second cam 44b having the larger outside diameters than the pressed member 41, and therefore, even though the spring 43 that presses the pressing member 42 to the upper side is a thin disk spring, the spring 43 can give a sufficient pressing force to the pressing member 42, when the pressed member 41 is sandwiched between the pressing member 42 and the second cam 44b, and therefore, a sufficient sandwiching force can be obtained.

Accordingly, the spring 43 can be made more compact in the height direction Z than the spring 33 shown in Fig. 6, and therefore, the holding mechanism 40 can be made more compact in the height direction Z than the holding mechanism 30 shown in Fig. 6.

By the above, and the holding mechanism 40 being provided at the intermediate position in the height direction Z of the shaft 9, the size in the height direction Z of the operation portion 5 can be made sufficiently small similarly to the present embodiment.

Further, hereinafter, another modification will be shown with use of Fig. 10. Fig. 10 is a view showing a modification in which the travel direction changing member is configured by a cylindrical member through which the pull wire at a position that is superimposed on the joystick is inserted, when the cylindrical member is planarly viewed from the height direction in the operation portion of Fig. 3.

In the present embodiment described above, the travel direction changing members are shown with the guide rollers 14u, 14d, 14r and 141 cited as the examples, but the travel direction changing members are not limited to them, and the travel direction changing member may be guide pipes 60u, 60d, 60r and 601 (the guide pipes 60r and 601 are not illustrated in Fig. 10) that are cylindrical members through which the pull wires 10u, 10d, 10r and 101 at positions that are superimposed on the joystick 15 are inserted, when the guide pipes are planarly viewed from the height direction Z in the operation portion 5.

The guide pipes 60u, 60d, 60r and 601 are respectively fixed into the operation portion 5 via fixing members 61. Note that the other positions where the guide pipes 60u, 60d, 60r and 601 are provided are the same as the guide rollers 14u, 14d, 14r and 141 in the present embodiment described above.

Further, when the guide pipes 60u, 60d, 60r and 601 are configured by flexible members, the guide pipes 60u, 60d, 60r and 601 deform even if the action range of the frame body 11 is large, and therefore, the guide pipes 60u, 60d, 60r and 601 guide the pull wires 10r, 10d, 10r and 101 by following movements of the pull wires 10u, 10d, 10r and 101.

Further, when the guide pipes 60u, 60d, 60r and 601 are configured by rigid members, the guide pipes 60u, 60d, 60r and 601 have similar functions to the function of the protection member such as the pipe 26 shown in Fig. 5.

Note that the guide pipes 60u, 60d, 60r and 601 may be each of a hybrid type in which a site in the vicinity of the frame body 11 is configured by a flexible member, and the other site is configured by a rigid member.

According to the configuration as above, the effect similar to the present embodiment described above can be obtained, in addition to which, the action range of the frame body 11 can be made large without increase of the resistance of the guide pipes 60u, 60d, 60r and 601.

Note that in the present embodiment, the guide pipes 60u, 60d, 60r and 601 may be provided in a region that is superimposed on the frame body 11 when the guide pipes are planarly viewed from the height direction Z.

Note that in the present embodiment described above, the configuration of bending the bending portion of the insertion portion of the endoscope is shown by being cited as an example, but the present invention is not limited thereto, and is also applicable to other insertion apparatuses such as a guide tube, various treatment instruments that do not have observation means, and a manipulator.

The present application is filed claiming the priority of Japanese Patent Application No. 2012-017219 filed in Japan on January 30, 2012, and the above described disclosure is incorporated by reference in the present description, claims and drawings.

## Claims

1. An insertion apparatus, comprising:
an insertion portion that is inserted into a subject, and is provided with a bending portion that bends in a plurality of directions;
an operation portion that is provided at a proximal end in an insertion direction of the insertion portion;
a pull member that is inserted through insides of the insertion portion and the operation portion, has a distal end in the insertion direction connected to the bending portion, and causes the bending portion to bend by pulling;
an operation input member that is provided in the operation portion, has a shaft that is raised in an intersection direction intersecting the insertion direction, an operation element provided at an upper end portion of the shaft and a connecting portion that is provided at a lower end portion of the shaft and to which a proximal end of the pull member in the insertion direction is connected in the operation portion, in which input of a pulling operation of the pull member is performed by the operation element and the shaft being tilted and the connecting portion being brought into action; and
a travel direction changing member that is provided outside an action range of the connecting portion that acts by being linked with tilt of the operation element and the shaft in the operation portion, and changes a travel direction of the pull member extended in the intersection direction from the connecting portion so that the pull member travels outside the action range of the connecting portion to a front side in the insertion direction.

2. The insertion apparatus according to claim 1, further comprising:
a drive member that is provided at a front side in the insertion direction from the operation input member in the operation portion, and generates a drive force that drives and bends the bending portion; and
a drive force transmitting member that is provided at the front side in the insertion direction from the operation input member in the operation portion, has a friction engaging portion that is frictionally engageable with the drive member by the pull member that is pulled by the operation input member, and transmits the drive force of the drive member to the pull member by the friction engaging portion being frictionally engaged with the drive member to give a pull assisting force to the pull member.

3. The insertion apparatus according to claim 2,
wherein the travel direction changing member is provided in a position at a same height as the drive member, in the intersection direction.

4. The insertion apparatus according to claim 1 or 2,
wherein the travel direction changing member is provided in a vicinity of the connecting portion, in the intersection direction.

5. The insertion apparatus according to claim 1 or 2,
wherein the shaft has a bent shape at an intermediate position in the intersection direction to avoid interference of the pull member at a time of the travel direction changing member being provided at the operation element side from the connecting portion, in the intersection direction.

6. The insertion apparatus according to claim 1 or 2,
wherein a holding mechanism that changeably holds a tilt angle of the operation element and the shaft is provided in a position at a side more opposite from the operation element than the connecting portion in the intersection direction, in the operation portion at a time of the travel direction changing member being provided at the operation element side from the connecting portion in the intersection direction.

7. The insertion apparatus according to claim 6,
wherein a protection member that protects the pull member is provided in a position where the pull member is superimposed on the operation input member in the operation portion, when the protection member is viewed from the intersection direction in a plan view.

8. The insertion apparatus according to claim 1 or 2,
wherein a holding mechanism that changeably holds a tilt angle of the operation element and the shaft is provided at an intermediate position of the shaft in the intersection direction, in the operation portion, at a time of the travel direction changing member being provided at a side more opposite from the operation element than the connecting portion, in the intersection direction.

9. The insertion apparatus according to claim 8,
wherein the connecting portion constitutes a protection member that protects the pull member.

10. The insertion apparatus according to claim 1 or 2,
wherein the travel direction changing member is a guide roller.

11. The insertion apparatus according to claim 1 or 2,
wherein the travel direction changing member is a cylindrical member into which the pull member is inserted in a position where the pull member is superimposed on the operation input member when viewed from the intersection direction in a plan view.

12. The insertion apparatus according to claim 11,
wherein the cylindrical member constitutes a protection member that protects the pull member in a position where the pull member is superimposed on the operation input member when viewed from the intersection direction in a plan view.
